Europäisches Patentamt

European Patent Office

Office européen des brevets

(11) Numéro de publication: **0 332 479**
**A1**

## DEMANDE DE BREVET EUROPEEN

(21) Numéro de dépôt: **89400315.1**

(22) Date de dépôt: **03.02.89**

(51) Int. Cl.4: **C 07 C 15/20**
C 07 C 5/00, C 07 C 5/32,
G 01 N 24/10, G 01 R 33/22

(30) Priorité: **12.02.88 FR 8801682**

(43) Date de publication de la demande:
**13.09.89 Bulletin 89/37**

(84) Etats contractants désignés: **DE ES GB IT**

(71) Demandeur: **THOMSON-CSF**
**51, Esplanade du Général de Gaulle**
**F-92800 Puteaux (FR)**

(72) Inventeur: **Michel, Philippe**
**THOMSON-CSF SCPI 19, avenue de Messine**
**F-75008 Paris (FR)**

**Moradpour, Alexandre**
**THOMSON-CSF SCPI 19, avenue de Messine**
**F-75008 Paris (FR)**

**Penven, Paul**
**THOMSON-CSF SCPI 19, avenue de Messine**
**F-75008 Paris (FR)**

(74) Mandataire: **Guérin, Michel et al**
**THOMSON-CSF SCPI**
**F-92045 PARIS LA DEFENSE CEDEX 67 (FR)**

(54) Procédé d'obtention de pérylènes polyalkyles, pérylènes obtenus par ce procédé, et matériaux organiques à propriétés RPE en dérivant.

(57) L'invention concerne un procédé de fabrication de pérylène polyalkyle consistant à faire subir une réaction de cyclodéshydrogénation par un métal alcalin au dérivé polyalkyle correspondant du 1,1'-dinaphtalène.

Elle concerne aussi le pérylène polyalkyle obtenu et le matériau organique qui en dérive et qui présente un spectre de résonance paramagnétique électronique.

FIG_1

1) Li(excès), THF 65°

2) O$_2$, température ambiante

EP 0 332 479 A1

**Description**

## PROCEDE D'OBTENTION DE PERYLENES POLYALKYLES, PERYLENES OBTENUS PAR CE PROCEDE ET MATERIAUX ORGANIQUES A PROPRIETES RPE EN DERIVANT

La présente invention concerne un nouveau procédé d'obtention de pérylènes polyalkyles. Elle concerne également les nouveaux produits obtenus par ce procédé : les pérylènes polyalkyles et les matériaux organiques qui en dérivent et qui possèdent des propriétés de résonance paramagnétique électronique remarquables.

Les sels de cations radicaux dérivés de certains hydrocarbures polyaromatiques condensés, tels le fluoranthène (FA) et le pérylène (PY) ont des propriétés de résonance paramagnétique électronique (RPE) remarquables. Pour (FA)$_2$ PF$_6$, par exemple, un signal RPE remarquablement étroit à la température ambiante a été observé.

Ce matériau possède une largeur pic-à-pic de la dérivée en champ du signal d'absorption qui n'est que de 15 milligauss. Ce signal est donc pratiquement 100 fois plus étroit que le signal RPE du radical diphénylpicrylhydrazyle (DPPH) utilisé habituellement comme marqueur de champ en RPE. Cependant, si l'absence d'hétéroatomes dans ces matériaux peut être favorable à l'étroitesse des largeurs de raies RPE, elle est en revanche un facteur qui entraîne une certaine instabilité thermique. Ainsi, par exemple, les sels préparés à partir du naphtalène ne sont stables qu'au-dessous de -40°C, alors que la largeur de la raie RPE correspondante est la plus étroite (4 milligauss) de toute cette série de matériaux.

L'étroitesse remarquable de ces raies de résonance paramagnétique électronique devrait permettre l'utilisation de ces matériaux pour la réalisation de magnétomètres de très haute sensibilité à condition que leur stabilité thermique dans le temps, largement insuffisante actuellement, soit notablement améliorée.

Pour pallier ces inconvénients, l'invention propose de nouveaux matériaux préparés à partir de pérylènes polyalkyles et pouvant remplacer avantageusement les matériaux organiques de l'art connu tels que ceux dérivant du fluoranthène dans la réalisation de sondes magnétiques RPE. Pour synthétiser ces nouveaux produits, il a fallu avoir recours à un procédé d'obtention original. Ce procédé met en oeuvre une réaction de cyclodéshydrogénation initiée par du lithium métallique, à partir des dérivés 1,1'-dinaphtalènes correspondants. Par rapport aux procédés de l'art connu, il a l'avantage de la simplicité et possède un rendement plus élevé.

L'invention a donc pour objet un procédé d'obtention de pérylène polyalkyle, caractérisé en ce qu'il consiste à faire subir une réaction de cyclodéshydrogénation par un métal alcalin au dérivé polyalkyle correspondant du 1,1'-dinaphtalène.

L'invention a aussi pour objet les pérylènes polysubstitués constitués du noyau pérylène tétra-substitué par un radical alkyle.

L'invention a encore pour objet des matériaux organiques présentant un spectre de résonance paramagnétique électronique, ces matériaux comprenant dans leur composition un pérylène polyalkyle tétra-substitué.

L'invention sera mieux comprise et d'autres avantages apparaîtront grâce à la description qui va suivre, donnée à titre non limitatif, accompagnée des dessins annexés parmi lesquels :

- La figure 1 est illustrative de la réaction chimique permettant d'obtenir le 3, 4, 9, 10-tétraméthyl pérylène ;
- la figure 2 est illustrative de la réaction chimique permettant d'obtenir le 2, 5, 8, 11-tétraméthyl pérylène.

A l'heure actuelle, un petit nombre seulement d'alkylpérylènes ont été synthétisés. Il s'agit des trois monométhyl régioisomères, du 1- (n butyl) - pérylène, du 1- et du 2- éthylpérylène, des 2,8-, 3,9- et 2,11- diméthylpérylènes. Les alkylpérylènes avec des niveaux de substitutions plus élevés sont rares et seul le 1, 2, 7 8-tétrahydrocyclopenta [cd ; lm] pérylène a été synthétisé avec un faible rendement(N. TANAKA, T. KASAI, Bulletin of the Chemical Society of Japan, 1981, vol. 54, p. 3026).

Selon l'art connu, les pérylènes monosubstitués sont obtenus par des réactions électrophiles (R. LAPOUYADE, J. PEREYRE et P. GARRIGUES, Comptes Rendus de l'Académie des Sciences de Paris, tome 303, p. 903, 1986). Par contre, la synthèse régiospécifique des dérivés disubstitués ne peut être obtenue que par des procédés multi-étapes impliquant la synthèse du noyau pérylène lui-même (H.E. ZIEGER, J.E. ROSENKRANZ, Journal of Organic Chemistry, vol. 29, p. 2469, 1964). Les synthèses connues du pérylène en tant que tel sont pénibles ou impliquent de nombreuses étapes pour un faible rendement (E. CLAR, Polycyclic Hydrocarbons, vol. II, Acad., Press, 1964, p. 24). La mise en oeuvre peut impliquer des réactions à température élevée du naphtalène, du 1,1'- dinaphtalène ou de son dérivé 2,2'- dihydroxy. On peut utiliser un procédé du type Ullmann (par exemple à partir de 1,8-diiodonaphtalènes) ou des réactions d'extrusion photochimique de dérivés à base d'étain préparés à partir des composés diiodo déjà cités. Des procédés d'annelation impliquant de multiples étapes et partant d'autres hydrocarbones polyaromatiques tels que l'anthracène ou le phénanthrène ont aussi été décrits.

La possibilité de préparer le pérylène à partir du 1,1'- dinaphtalène par action du lithium métallique a déjà été mentionnée depuis longtemps (H. GILMAN, C.G. BRANNEN, Journal of the American Chemical Society, 1949, vol. 71, p. 657). Cependant, ce procédé s'est limité à la préparation du simple pérylène.

Selon l'invention, le procédé général de synthèse comprend les opérations suivantes. Une suspension de poudre d'un métal alcalin dans un solvant inerte est introduite dans un appareil à reflux et est chauffée et agitée vigoureusement sous une atmosphère neutre. A cette suspension, on ajoute du 1,1′ -dinaphtalène substitué par le radical alkyle désiré pour le pérylène polyalkyl à obtenir. L'ensemble est chauffé à reflux. Un changement de couleur du mélange réactionnel, correspondant vraisemblablement à la formation de radicaux-anions intermédiaires, peut être observé au bout d'un certain temps. On laisse refroidir le mélange jusqu'à la température ambiante, puis de l'oxygène est soufflé dans le flacon le contenant. Une décoloration est observée. L'excès de métal alcalin est éliminé par filtration et lavé par une petite quantité du solvant réactionnel. Le solvant est ensuite évaporé sous vide et une masse brune est recueillie et chromatographiée. Les pérylènes substitués obtenus sont recristallisés à partir du xylène.

Pour illustrer le procédé général de synthèse, quelques exemples de fabrication de pérylènes polyalkyles, donnés à titre non limitatifs, vont être décrits. De même, dans ces exemples, le métal alcalin utilisé sera le lithium.

Exemple 1: synthèse du 3, 4, 9, 10- tétraméthyl pérylène.

Il s'agit d'abord de préparer le 1,1′- dinaphtalène substitué correspondant, en l'occurrence le 4,4′, 5,5′-tétraméthyl 1,1′- dinaphtalène. Ce produit est obtenu, avec un rendement de 32%, à partir du 1,8-diméthylnaphtalène disponible chez la Société MERCK, par couplage oxydant avec le tétraacétate de plomb, selon un procédé de l'art connu (A. McKILLOP, A.G. TURRELL, D.W. YOUNG, E.C. TAYLOR, Journal of the American Chemical Society, 1980, Vol. 102, p. 6504).

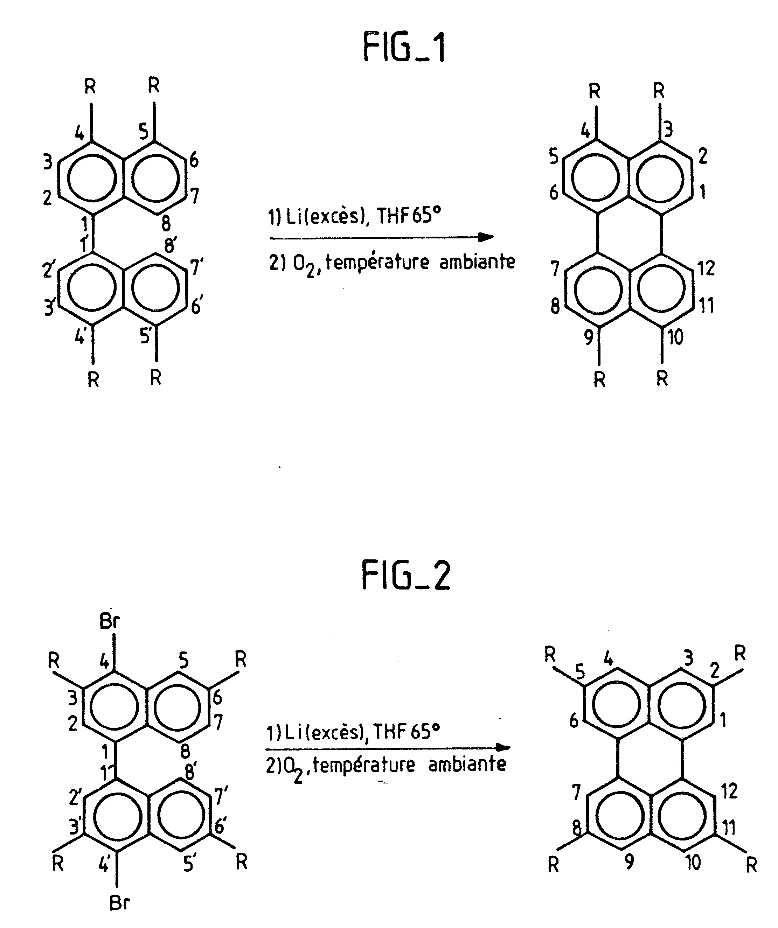

On applique ensuite le procédé général de synthèse décrit plus haut pour réaliser la réaction chimique illustrée par la figure 1. On prépare une suspension de poudre de lithium dans un solvant inerte, par exemple du tétrahydrofuranne (THF). Les proportions peuvent être les suivantes : 1,5 g soit 220 millimoles de lithium pour 170 ml de THF. On peut utiliser de l'argon comme atmosphère neutre dans l'appareil à reflux. A ce mélange, on ajoute 3 millimoles de 4,4′, 5,5′-tétraméthyl 1,1′- dinaphtalène sous forme solide. Le chauffage à reflux est maintenu à 65°C pendant 5 heures, durée correspondant à la disparition complète du 1,1′ - dinaphtalène initialement engagé. La couleur du mélange réactionnel tourne au bleu après les 3 premières heures de chauffage. Après refroidissement et injection d'oxygène, la couleur du mélange tourne au jaune. L'excès de lithium est éliminé et le solvant est évaporé. La chromatographie sur gel de silice est effectuée avec le dichlorométhane comme éluant. On obtient le 3, 4, 9, 10-tétraméthyl pérylène par recristallisation à partir du xylène. Le rendement de l'opération est de 40%.

Exemple 2: synthèse du 2, 5, 8, 11- tétraméthyl pérylène.

Il s'agit d'abord de préparer le 1,1′- dinaphtalène substitué correspondant, en l'occurrence le 4,4′-dibromo -3,3′, 6,6′- tétraméthyl - 1,1′- dinaphtalène. Ce produit est obtenu, avec un rendement de 32%, à partir du 1- bromo - 2,7 -diméthylnaphtalène (J.

WOLINSKA-MOCYDLARZ, P. CANONNE, L.C. LEITCH, Synthesis, 1974, p. 566) par couplage avec le trifluoro-acétate de thallium III (A. McKILLOP, A.G. TURRELL, D.W. YOUNG, E.C. TAYLOR, Journal of the American Chemical Society, 1980, Vol. 102, p. 6504).

On applique ensuite le procédé général de synthèse décrit plus haut pour réaliser la réaction chimique illustrée par la figure 2. Comme pour l'exemple 1, le mélange contenant de la poudre de lithium en suspension peut être préparée avec du THF comme solvant, dans les mêmes proportions. On peut utiliser de l'argon comme atmosphère neutre dans l'appareil à reflux. A ce mélange, on ajoute 3 millimoles de 4,4′- dibromo-3,3′, 6,6′-tétraméthyl- 1,1′- dinaphtalène sous forme solide. Le chauffage à reflux est maintenu à 65°C pendant 5 heures jusqu'à disparition du 1,1′ - dinaphtalène initial. La couleur du mélange réactionnel tourne au violet foncé après les 3 premières heures de chauffage. Après refroidissement et injection d'oxygène, la couleur du mélange tourne au rouge. L'excès de lithium est éliminé et le solvant est évaporé. La chromatographie sur gel de silice est effectuée avec un mélange de toluène et de cyclohexane (respectivement 15 et 85% en volume) comme éluant. On obtient le 2, 5, 8, 11- tétraméthyl pérylène par recristallisation à partir du xylène. Le rendement de l'opération est de 36%.

Pour les exemples 1 et 2, on a utilisé la technique de chromatographie sur silice dite flash-chromatography (W.CLARK STILL, M.KAHN, A. MITRA, Journal of Organic Chemistry, 1978, vol.43, p.2923).

Le procédé selon l'invention, en plus de la relative simplicité de sa mise en oeuvre par rapport aux procédés cités plus haut, a aussi l'avantage de présenter un rendement élevé (respectivement 40 et 36% pour les exemples 1 et 2).

Il entre dans le cadre de l'invention d'élaborer d'autres pérylènes tétra-substitués par un radical alkyle que ceux décrits dans les exemples 1 et 2. Il suffit pour cela d'obtenir le dérivé polyalkyle correspondant du 1,1′- dinaphtalène en se référant au procédé de l'art connu cité plus haut.

Les nouvelles molécules élaborées par le procédé selon l'invention peuvent être utilisées pour obtenir des matériaux ayant des propriétés de résonance paramagnétique électronique remarquables. Ces matériaux peuvent être obtenus par la technique bien connue de l'électrocristallisation. Par exemple, l'oxydation anodique du 3, 4, 9, 10-tétraméthylpérylène (TMP) peut être effectuée en solution ($3.10^{-5}$M) dans du dichlorométhane (anhydre, en absence d'oxygène) en présence d'hexafluorophosphate de tétrabutylammonium ($5.10^{-4}$M) à -30°C. Les intensités utilisées au cours de l'opération sont de quelques $\mu$A. Le matériau isolé correspond à la formule :
$[(TMP)_2 \, PF_6, \, n \, CH_2Cl_2]$ avec $0 < n \leq 2$

On peut se débarrasser du dichlorométhane $CH_2Cl_2$ par chauffage prolongé à plus de 80°C, sous vide.

Ce matériau possède une largeur de raie RPE de 16 milligauss à température ambiante. Il a l'avantage d'être stable jusqu'à plus de 80°C et d'être stable à l'air.

On peut remplacer l'anion PF⁻₆ par AsF⁻₆ en partant de l'hexafluoroarsénate de tétrabutylammonium et obtenir un matériau ayant des propriétés RPE voisines.

Les nouveaux matériaux obtenus, présentant un spectre de résonance paramagnétique électronique remarquable et étant stable à la température ambiante, remplaceront avantageusement les matériaux à base de fluoranthène dans l'élaboration des gaussmètres ou magnétomètres.

## Revendications

1. Procédé d'obtention de pérylène polyalkyle, caractérisé en ce qu'il consiste à faire subir une réaction de cyclodéshydrogénation par un métal alcalin au dérivé polyalkyle correspondant du 1,1'- dinaphtalène.

2. Procédé selon la revendication 1, caractérisé en ce que la réaction de cyclodéshydrogénation comprend les étapes suivantes :
- 1ère étape : chauffage, sous atmosphère neutre, d'un mélange constitué d'un solvant et d'une suspension de poudre d'un métal alcalin,
- 2ème étape : ajout à ce mélange de 1,1'-dinaphtalène substitué par le radical alkyle désiré pour ledit pérylène polyalkyle et continuation du chauffage pendant une durée déterminée,
- 3ème étape : injection d'oxygène sur le mélange constitué à la 2ème étape, après le refroidissement de ce mélange,

- 4ème étape : élimination du métal alcalin en excès et évaporation du solvant,
- 5ème étape : obtention du pérylène polyalkyle par recristallisation.

3. Procédé selon l'une des revendications 1 ou 2, caractérisé en ce que ledit métal alcalin est du lithium.

4. Pérylène polyalkyle, caractérisé en ce qu'il est constitué de pérylène tétra-substitué par un radical alkyle.

5. Pérylène polyalkyle selon la revendication 4, caractérisé en ce que la substitution par le radical alkyle intervient en position 3, 4, 9 et 10.

6. Pérylène polyalkyle selon la revendication 4, caractérisé en ce que la substitution par le radical alkyle intervient en position 2, 5, 8 et 11.

7. Pérylène polyalkyle selon l'une des revendications 5 ou 6, caractérisé en ce que ledit radical alkyle est le méthyle.

8. Matériau organique présentant un spectre de résonance paramagnétique électronique, caractérisé en ce qu'il comprend dans sa composition un pérylène polyalkyle selon l'une des revendications 4 à 7.

9. Matériau organique selon la revendication 8, caractérisé en ce que sa composition comprend essentiellement de l'hexafluorophosphate de 3, 4, 9, 10- tétraméthyl pérylène.

10. Matériau organique selon la revendication 8, caractérisé en ce que sa composition comprend essentiellement de l'hexafluoroarsénate de 3, 4, 9, 10- tétraméthyl pérylène.

# FIG_1

1) Li(excès), THF 65°

2) O$_2$, température ambiante

# FIG_2

1) Li(excès), THF 65°

2) O$_2$, température ambiante

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | CHEMICAL ABSTRACTS<br>tome 69, no. 19, 4 novembre 1968,<br>abrégé no. 76169f, Columbus, Ohio, US;<br>S.P. SOLODOVNIKOV et al.:"The formation<br>of perylene by the interaction between<br>metallic potassium and 1,1-binaphthyl<br>in 1,2-dimethoxyethane."; & Izv. Akad.<br>Nauk SSSR. Ser. Khim.1968 (2), 442-4<br>--- | 1,8 | C 07 C   15/20<br>C 07 C   5/00<br>C 07 C   5/32<br>G 01 N   24/10<br>G 01 R   33/22 |
| A | BEILSTEINS HANDBUCH DER ORGANISCHEN<br>CHEMIE<br>4 édition, Imprimerie Springer, Berlin,<br>tome 5, 3 supplément, 4 partie * page<br>2538, paragraphe 6 *<br>--- | 1 | |
| A | US-A-3 132 187  (TURETZKY)<br>* document en entier *<br>--- | 1 | |
| A | DE-C-  469 553  (COMPAGNIE NATIONALE DE<br>MATIERES COLORANTES ET DE PRODUITS<br>CHIMIQUES)<br>* revendication *<br>--- | 1 | |
| A | DE-C-  638 002  (I.G. FARBENINDUSTRIE)<br>* exemples *<br>--- | 1 | |
| A | DE-B-2 640 471  (ENTREPRISE DE<br>RECHERCHES ET D'ACTIVITES PETROLIERES)<br>* revendications *<br>----- | 1 | |

DOMAINES TECHNIQUES
RECHERCHES (Int. Cl.4)

C 07 C   15/00
C 07 C   5/00
G 01 N   24/00

Le présent rapport a été établi pour toutes les revendications

| Lieu de la recherche | Date d'achèvement de la recherche | Examinateur |
|---|---|---|
| BERLIN | 06-04-1989 | PROBERT C.L. |